# EUROPEAN PATENT APPLICATION

(11) **EP 3 973 802 A1**
(43) Date of publication of application: **30.03.2022**
(21) Application number: 19930058.3
(22) Date of filing: 22.05.2019
(51) Int. Cl.: A24F 47/00

(54) **ASPIRATION DEVICE, ASPIRATION EXPERIENCE PROVISION SYSTEM, METHOD, AND PROGRAM**

(71) Applicant: Japan Tobacco Inc., Tokyo 105-6927 (JP)
(72) Inventor: TATSUTA, Nobuhiro, Tokyo 130-8603 (JP); FUJITA, Ryoji, Tokyo 130-8603 (JP); TEZUKA, Hiroshi, Tokyo 130-8603 (JP)
(74) Representative: Hoffmann Eitle
(86) International application number: PCT/JP2019/020329
(87) International publication number: WO 2020/235062

(57) **Abstract**

The present invention provides an aspiration device that makes it possible to enhance the quality of an aspiration experience provided to a user. There is provided an aspiration device in which an aspiration article that accommodates a flavor source is detachably mounted. This aspiration device is characterized in that: the aspiration article is provided with a flavor designation part that indicates at least the type of the flavor source; and the aspiration device is provided with a holding part that holds the flavor source, a sensing operation part that senses the aspiration article and interacts with the flavor designation part, a control unit that discriminates the type of flavor source through the sensing operation part, and a notification unit that issues notification of a state that corresponds to the result of discrimination by the control unit.

## Description

### TECHNICAL FIELD

The present disclosure relates to an inhaler, and an inhalation experience providing system, a method, and a program.

### BACKGROUND ART

As an electronic device, an inhaler which generates an inhaled component, such as flavor-added aerosol, has been known. In a puff action performed by a user, a flavor-added inhalation article (This is also referred to as a refill.) is attached to an inhaler, usually. Further, refills, to which various kinds of flavors have been added, have been developed for providing a user with inhalation experience suitable to user's preference. In this regard, the inhalation experience refers to, for example, experience that is provided as a result of puff of aerosol, and at least one of five senses of a user is stimulated thereby.

For example, there are types in flavors, such as a mint flavor, a coffee flavor, a menthol flavor, and so on, and they are also refereed to as flavours. Each flavour has a unique concept for providing a user with a good flavor. A user will select a flavour according to user's preference, and install it to an inhaler.

A method for detecting an inhalation article, when the article is attached to an inhaler, by using a sensor installed in the inhaler has been known. For example, Patent Literature 1 discloses a technique to detect attaching of an inhalation article by detecting light reflected from the inhalation article by using an optical sensor. Further, each of Patent Literatures 2 and 3 discloses a technique to detect thermal proximity between an inhalation article and a heating element of an inhaler by using a proximity sensor.

### CITATION LIST

### PATENT LITERATURE

PTL 1: Japanese Patent Application Public Disclosure No. H07-184627
PTL 2: Japanese Patent Application Public Disclosure No. 2011-515093
PTL 3: Japanese Patent Application Public Disclosure No. 2015-506170

### SUMMARY OF INVENTION

### TECHNICAL PROBLEM

It is desirable to provide a user with a flavor that is suitable to user's preference, and, in addition thereto, provide the user with puff environment that allows the user to taste the flavor satisfactory.

The present disclosure has been achieved in view of the above matters. That is, an object of the present disclosure is to provide an inhaler which can further improve the quality of inhalation experience provided to a user. Specifically, one of objects is to provide puff environment (for example, an atmosphere at the time of puff) corresponding to a concept of a flavor that is to be inhaled by a user, provide an inhaler that can be operated to correspond to such an puff environment, and provide more appropriate inhalation experience.

In addition, a different object of the present disclosure is to take points of view of energy conservation and environmental protection into consideration when providing puff environment corresponding to a concept of a flavor. Specifically, one of objects is to optimize operation of the inhaler to thereby reduce the frequency of discarding of inhalers, batteries, inhalation articles, and so on by extending the spans of life thereof, and provide an environmentally friendly inhaler by preventing unnecessary waste of inhaled component sources.

### SOLUTION TO PROBLEM

In a first aspect, an inhaler, to which an inhalation article including a flavor source is detachably attached, is provided. Regarding the inhaler, the inhalation article comprises a flavor indication unit which represents at least the type of the flavor source, and the inhaler is characterized in that it comprises: a holder for holding the flavor source; a detection function unit which detects the inhalation article and interacts with the flavor indication unit; a controller which judges, through the detection function unit, the type of the flavor source; and a notifier which performs notification in a mode corresponding to a result of judgment performed by the controller.

An inhaler in a second aspect comprises the inhaler in the first aspect, and the mode comprises emission of light corresponding to the judged flavor-source type.

An inhaler in a third aspect comprises the inhaler in the first aspect or the second aspect, and the mode comprises generation of vibration corresponding to the judged flavor-source type.

An inhaler in a fourth aspect comprises the inhaler in any one of those in the first aspect to the third aspect, and the mode comprises generation of a sound corresponding to the judged flavor-source type.

An inhaler in a fifth aspect comprises the inhaler in any one of those in the first aspect to the fourth aspect, and further comprises: a vent for making air flow therein, and a vent adjusting mechanism which can adjust an opening area of the vent; and the controller is configured to make the vent adjusting mechanism adjust the opening area of the vent based on the judged flavor-source type.

An inhaler in a sixth aspect comprises the inhaler in any one of those in the first aspect to the fifth aspect, and further comprises a power button; and the controller is configured to judge the flavor-source type in response to pressing of the power button by a user.

An inhaler in a seventh aspect comprises the inhaler in any one of those in the first aspect to the fifth aspect, and further comprises a sensor which can detect puff action; and the controller is configured to judge the flavor-source type in response to detection of puff action performed by a user.

An inhaler in a eighth aspect comprises the inhaler in any one of those in the first aspect to the seventh aspect, and further comprises an electric power source part and a heater; and the controller is configured to periodically judge the flavor-source type, during the time when electric power is being supplied from the electric power source part to the heater.

An inhaler in a ninth aspect comprises the inhaler in the eighth aspect, and the controller is configured to stop supplying of electric power from the electric power source part to the heater when the judgment has ended in failure.

An inhaler in a tenth aspect comprises the inhaler in any one of those in the first aspect to the ninth aspect, and, in the inhaler, the detection function unit is installed in a position close to an opening into which the inhalation article is inserted.

An inhaler in a eleventh aspect comprises the inhaler in any one of those in the first aspect to the tenth aspect, and further comprises an external connection terminal; and the mode is changed based on an instruction from an external input device connected via the external connection terminal.

An inhaler in a twelfth aspect comprises the inhaler in any one of those in the first aspect to the eleventh aspect, and further comprises a communication module; and the communication module is configured to transmit information of the judged flavor-source type to a network.

In a thirteenth aspect, an inhalation experience providing system comprising the inhaler in the twelfth aspect is provided. The inhalation experience providing system further comprises an external device which is connected to the inhaler via the network; and, when information of the flavor-source type is received by the external device, the external device is configured to generate an aroma corresponding to the received flavor-source type.

In a fourteenth aspect, an inhalation experience providing system comprising the inhaler in the twelfth aspect is provided. The inhalation experience providing system further comprises an external device which is connected to the inhaler via the network; and, when information of the flavor-source type is received by the external device, the external device is configured to generate a sound corresponding to the received flavor-source type.

In a fifteenth aspect, a method for operating an inhaler is provided. In the method, an inhalation article including a flavor source is attached to the inhaler, and the method comprises: detecting the inhalation article, and interacting with a flavor indication unit which is provided in the inhalation article and represents at least the type of the flavor source; judging, according to the interaction, the type of the flavor source; and performing notification in a predetermined mode corresponding to the judged flavor-source type.

A method in a sixteenth aspect comprises the method in the fifteenth aspect, and further comprises accepting pressing, by a user, of a power button; and the detecting and interacting step is performed in response to the accepting step.

A method in a seventeenth aspect comprises the method in the fifteenth aspect, and further comprises detecting puff action performed by a user; and the judging step is performed in response to the detecting step.

A method in an eighteenth aspect comprises one of the methods in the fifteenth aspect to the seventeenth aspect, and the judging step is periodically performed during the time when electric power is supplied to a heater from an electric power source part included in the inhaler.

A method in a nineteenth aspect comprises the method in the eighteenth aspect, and further comprises stopping supplying of electric power to the heater from the electric power source part, in the case that the judgment has ended in failure.

In a twentieth aspect, a program which makes the inhaler perform the method recited in any one of the methods in the fifteenth aspect to the nineteenth viewpoint.

### BRIEF DESCRIPTION OF DRAWINGS

Fig. 1A is an overall perspective view of an inhaler according to a first embodiment.
Fig. 1B is an overall perspective view of the inhaler in a state that it is holding an inhalation article, according to the first embodiment.
Fig. 2 is a cross-section view of an inhalation article.
Fig. 3 is a cross-section view, along arrows 3-3 shown in Fig. 1A, of the inhaler.
Fig. 4 is a schematic block diagram of a construction of the inhaler according to the first embodiment.
Fig. 5 is a schematic flow chart of operation of the inhaler according to the first embodiment.
Fig. 6 is an example of application of the inhaler according to the first embodiment to an inhalation experience providing system.
Fig. 7 is a sequence diagram showing operation of the inhalation experience providing system in Fig. 6.
Fig. 8 is a schematic block diagram of a construction of an inhaler according to a second embodiment.

### DESCRIPTION OF EMBODIMENTS

In the following description, inhalers according to embodiments of the present disclosure will be explained in detail with reference to the attached figures. It should be reminded that, although the inhalers in the embodiments of the present disclosure include an electronic cigarette and a nebulizer, the inhalers are not limited to those explained above. Especially, they may include various inhalers for generating aerosol or flavor-added aerosol inhaled by users. Further, the generated inhaled component sources include invisible vapor, in addition to aerosol.

In the attached figures, a reference number assigned to one component is assigned to the other component if the other component is the same as or similar to the one component, and, in the explanation of respective embodiments, explanation of a component, that is the same as or similar to the other component, may be omitted for avoiding overlapping explanation. Also, a characteristic shown in each embodiment can be applied to the other embodiment if there is no contradiction between the embodiments. Further, the figures are drawn in a schematic manner, so that sizes, ratios, and so on therein may be different from actual sizes, ratios, and so on. Further, the figures may include a figure which includes a part in which relationship and ratios between sizes are different from those in a corresponding part in a different figure.

### < First Embodiment >

### (1-1) Basic Construction of Inhaler

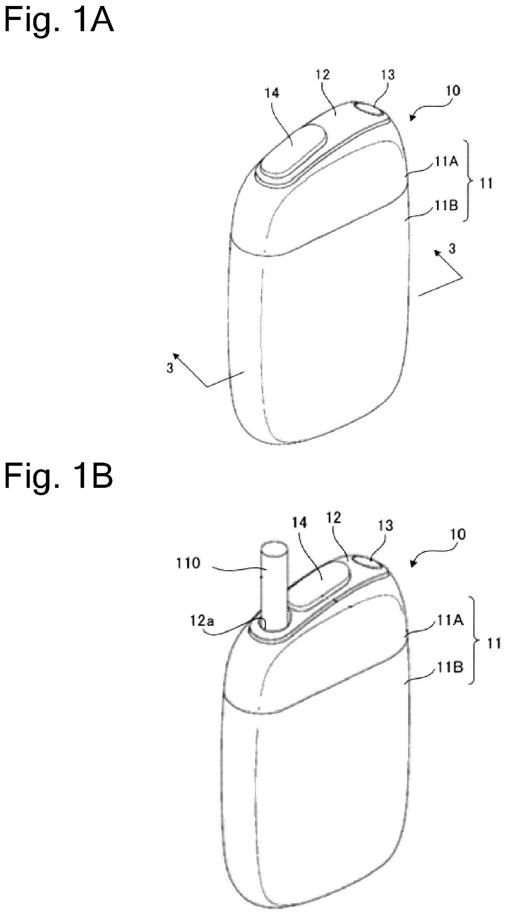

Fig. 1A is an overall perspective view of an inhaler 10 according to a first embodiment. Fig. 1B is an overall perspective view of the inhaler 10 in a state that it is holding an aerosol generation base-material, according to the first embodiment. In the present embodiment, to the inhaler 10, an aerosol generation base-material such as an inhalation article 110 or the like, which comprises an aerosol source and a flavor generation base-material such as a filling article comprising a flavor source or the like, is attached in an attachable/detachable manner. Further, it is constructed to generate aerosol including flavor by heating an attached inhalation article.

As would be understood by a person skilled in the art, the aerosol generation base-material is an example of the inhalation article 110 (hereinafter, the aerosol generation base-material may be collectively referred to as an inhalation article). An aerosol source included in an aerosol generation base-material may be solid or liquid. The aerosol source may be liquid such as polyhydric alcohol, such as glycerin or propylene glycol, or water, or the like, for example. The aerosol source may comprise a tobacco raw material or an extract originated from a tobacco raw material, which releases a fragrance-inhaling-taste component when it is heated. In the case that the inhaler 10 is an inhaler for medical use, such as a nebulizer or the like, the aerosol source may comprise a medicine that is to be sucked by a patient. The aerosol generation base-material may not comprise a flavor source, depending on intended use thereof.

As shown in Figs. 1A and 1B, the inhaler 10 comprises a top housing 11A, a bottom housing 11B, a cover 12, a power button 13, and a lid part 14. The outermost housing 11 of the inhaler 10 is constructed as a result that the top housing 11A and the bottom housing 11B are connected to each other. The housing 11 may have a size that fits in a hand of a user. In the above case, when a user uses the inhaler 10, the user can hold the inhaler 10 by a user's hand, and suck aerosol.

The top housing 11A comprises an opening (not shown in the figures), and the cover 12 is coupled to the top housing 11A to close the opening. As shown in Fig. 1B, the cover 12 has an opening 12a into which the inhalation article 110 can be inserted. The lid part 14 is constructed to open/close the opening 12a of the cover 12. Specifically, the lid part 14 is attached to the cover 12, and constructed to be able to move, along a surface of the cover 12, between a first position for closing the opening 12a and a second position for opening the opening 12a.

The power button 13 is used for switching between an ON state and an OFF state of operation of the inhaler 10. For example, in a state that the inhalation article 110 has been inserted in the opening 12a as shown in Fig. 1B, a user can make the inhalation article 110 be heated without combustion thereof, by pressing the power button 13 to thereby supply electric power from the electric power source part 20 to the heater 40. As a result that the inhalation article 110 is heated, aerosol is generated from an aerosol source included in the inhalation article 110, and flavor in a flavor source is taken in the aerosol. A user can suck aerosol including flavor, by performing puff action applied to a part of the inhalation article 110 projecting from the inhaler 10 (the part shown in Fig. 1B). In this specification, the direction of insertion of an aerosol generation base-material, such as the inhalation article 110 or the like, into the opening 12a is referred to as a longitudinal direction of the inhaler 10.

The construction of the inhaler 10 shown in Fig. 1A and Fig. 1B is a mere example of the construction of the inhaler according to the present disclosure. The inhaler 10 according to the present disclosure can be constructed to have any of various forms that allows generation of aerosol by heating the inhalation article 110 (aerosol generation base-material) including an aerosol source, and puff of the generated aerosol by a user.

### (1-2) Construction of Inhalation Article (Aerosol Generation Base-Material)

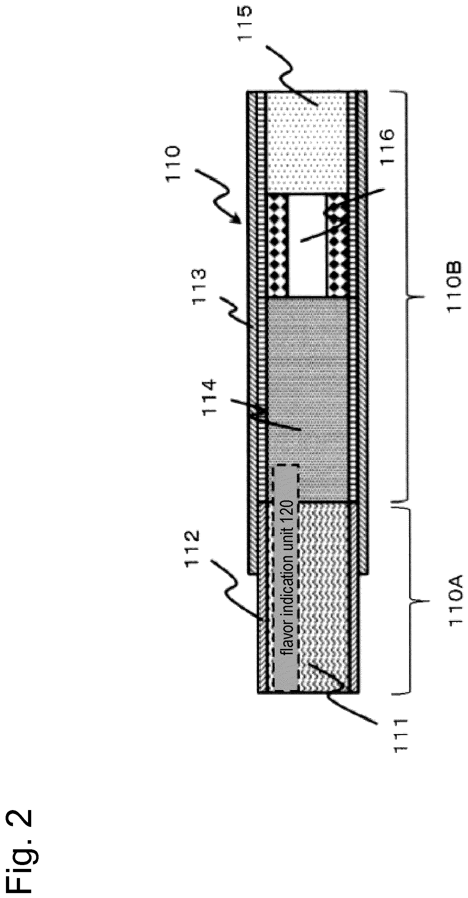

Next, the construction of the inhalation article 110, which is an aerosol generation base-material comprising a flavor source and is used in relation to the inhaler 10 according to the present embodiment, will be explained. Fig. 2 is a cross-section view of the inhalation article 110. In Fig. 2, the inhalation article 110 comprises a base-material part 110A, which comprises a filling article 111 (this corresponds to an example of the flavor generation base-material) and first rolling paper 112 by which the filling article 111 is wound, and a puff opening part 110B which forms an end part opposite to the base-material part 110A. The base-material part 110A and the puff opening part 110B are connected by second rolling paper 113 which is different from the first rolling paper 112. In this regard, it is possible to connect the base-material part 110A and the puff opening part 110B by using the first rolling paper 112, i.e., by omitting the second rolling paper 113.

The puff opening part 110B in Fig. 2 comprises a paper tube part 114, a filter 115, and a hollow segment part 116 positioned between the paper tube part 114 and the filter 115. For example, the hollow segment part 116 comprises a filling layer including one or plural hollow channels, and a plug wrapper for covering the filling layer. Since the density of filled fibers in the filling layer is high, air and aerosol flows through the hollow channel only, and almost no air and aerosol flows through the filling layer, when puff action is performed. Regarding the inhalation article 110, if it is desired to lower a decrease in the quantity of delivery of aerosol due to filtering of the aerosol components in the filter 115, shortening the length of the filter 115 and replacing that part by the hollow segment part 116 will be effective for increasing the quantity of delivery of the aerosol.

In the embodiment shown in Fig. 2, the puff opening part 110B comprises three segments. However, in a different embodiment, the puff opening part 110B may be constructed by using one or two segments, or may be constructed by using four or more segments. For example, it is possible to omit the hollow segment part 116, and form the puff opening part 110B by arranging the paper tube part 114 and the filter 115 adjacent to each other.

In the embodiment shown in Fig. 2, regarding the longitudinal-direction length of the inhalation article 110, it is preferable to set it to 40-90 mm, more preferable to set it to 50-75 mm, and still more preferably to set it to 50-60 mm. Regarding the circumference of the inhalation article 110, it is preferable to set it to 15-25 mm, more preferable to set it to 17-24 mm, and still more preferably to set it to 20-22 mm. Further, regarding the inhalation article 110, the length of the base-material part 110A may be 20 mm, the length of the first rolling paper 112 may be 20 mm, the length of the hollow segment part 116 may be 8 mm, and the length of the filter 115 may be 7 mm. The length of each of the above segments may be changed appropriately, according to suitability to manufacture, required quality, and so on.

The filling article 111 in the inhalation article 110 may comprise an aerosol source which generates aerosol when it is heated at predetermined temperature. The kind of the aerosol source is not specifically limited, and extracted material and/or components thereof, that are obtained from various natural products, may be selected as an aerosol source according to intended use. Glycerin, propylene glycol, triacetin, 1,3-butanediol, and a mixture thereof, for example, can be listed as aerosol sources. The aerosol source content of the filling article 111 is not specifically limited; and, in view of generation of sufficient quantity of aerosol and satisfactory addition of fragrance inhaling taste, the aerosol source content is usually equal to or greater than 5 weight percent, and is preferably equal to or greater than 10 weight percent, and is usually equal to or less than 50 weight percent, and is preferably equal to or less than 20 weight percent.

The filling article 111 in the inhalation article 110 may comprise shredded tobacco as a flavor source. The material of shredded tobacco is not specifically limited, and publicly known material such as a lamina, a stem, and so on may be used as the material. The range of the content of the filling article 111 in the inhalation article 110, in the case that the circumference is 22 mm and the length is 20 mm, is, for example, 200-400 mg, and is preferably 250-320 mg. The water content of the filling article 111 is, for example, 8-18 weight percent, and is preferably 10-16 weight percent. In the case that the water content is that explained above, occurrence of staining at the time of rolling is suppressed, and suitability to rolling at the time of manufacture of the base-material part 110A is made satisfactory. There is no special limitation with respect to the size, the preparation method, and so on of the shredded tobacco used as the filling article 111. For example, dried tobacco leaves cut into pieces, each having the width of 0.8-1.2 mm, may be used. Alternatively, dried tobacco leaves are crushed and uniformized to become particles, regarding which the average particle size is approximately 20-200 µm, and the particles are processed to become a sheet, and the sheet cut into pieces, each having the width of 0.8-1.2 mm, may be used. Further, the above sheet formed via the sheet process may be processed to gather it without cutting it, and the gathered sheet may be used as the filling article 111.

In the present embodiment, each sheet of the first and second rolling paper 112 and 113 in the inhalation article 110 may be constructed by use of base paper which has the basis weight of, for example, 20-65 gsm, preferably, 25-45 gsm. The thickness of each sheet of the first and second rolling paper 112 and 113 is not specifically limited; however, in view of rigidness, gas permeability, and easiness of adjustment at the time of paper manufacture, the thickness is set to 10-100 µm, preferably, set to 20-75 µm, and, more preferably, set to 30-50 µm.

Filler may be included in the rolling paper 112 and 113 in the inhalation article 110. The filler content may be equal to or greater than 10 weight percent and less than 60 weight percent, and, may preferably be 15-45 weigh percent, with respect to the total weight of the first rolling paper 112 and the second rolling paper 113. It is preferable that the filler be 15-45 weight percent, with respect to a preferable range of basis weight (25-45 gsm). For example, calcium carbonate, titanium dioxide, kaolin, and so on may be used as filler. Paper including filler such as that explained above presents a white color that is preferable in view of appearance of paper used as rolling paper of the inhalation article 110, and is able to keep its whiteness permanently. By including a large quantity of filler such as that explained above, the ISO whiteness of rolling paper can be raised to 83 % or more, for example. Further, in view of practicality in terms of use of it as rolling paper in the inhalation article 110, it is preferable that the first rolling paper 112 and the second rolling paper 113 have the tensile strength of 8N/15mm or more. The tensile strength can be increased by reducing the filler content. Specifically, it can be increased by reducing the filler content to that less than the upper limit of the filler content that has been shown with respect to each range of the basis weight illustrated in the above description.

In addition, in Fig. 2, the inhalation article 110 is provided with a flavor indication unit 120. The flavor indication unit 120 shows, at least, the type of a flavor source included in the inhalation article 110. For example, the part of the flavor indication unit 120 is constructed to have a shape or a material according to a flavor-source type. That is, for example, it is possible to construct it in such a manner that the mode of contact (for example, the area of contact) between a detection function unit 90 of the inhaler 10 and the flavor indication unit 120, when the inhalation article 110 is inserted into the inhaler 10 is set to correspond to each of flavor-source types.

It should be reminded that, although Fig. 2 shows a construction wherein the flavor indication unit 120 is installed on the tip of the base-material part 110A side and the first rolling paper 112 and/or the second rolling paper 113 in the inhalation article 110, the construction is not limited to the above construction. Any arrangement of the flavor indication unit 120 may be acceptable if the flavor indication unit 120 is positioned in a position that can be aligned with the position of the detection function unit 90 of the inhaler 10.

### (1-3) Internal Construction of Inhaler

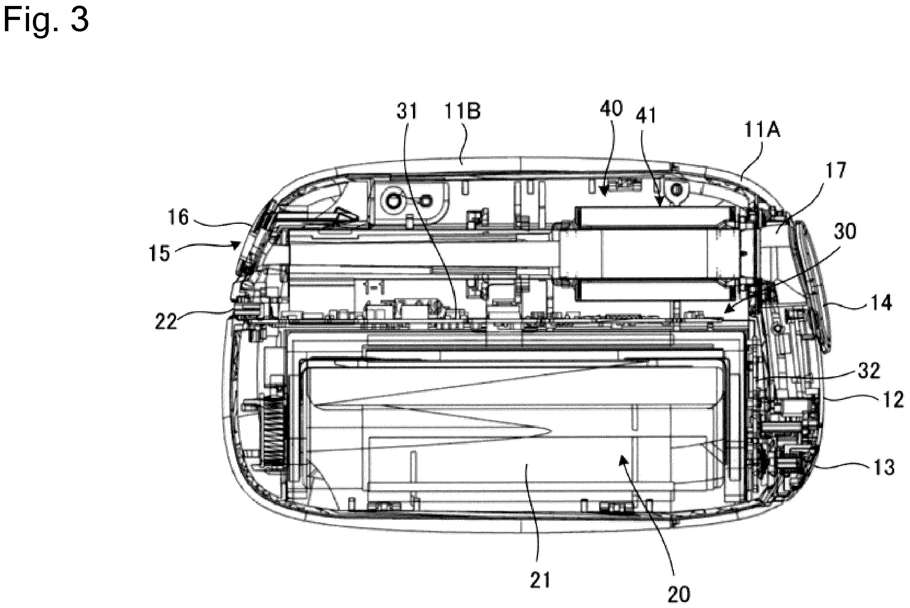

Next, an internal construction of the inhaler 10 shown in Fig. 1A and Fig. 1B will be explained. Fig. 3 is a cross-section view along arrows 3-3 shown in Fig. 1A. As shown in Fig. 3, the inhaler 10 comprises, in the inner space of the housing 11 (11A and 11B), an electric power source part 20, a circuit unit 30, and a heater 40. The circuit unit 30 may comprise a first circuit board 31 and a second circuit board 32 which is electrically connected to the first circuit board 31. For example, the first circuit board 31 may be arranged in such a manner that it extends in the longitudinal direction as shown in the figure. That is, the electric power source part 20 and the heater 40 are partitioned by the first circuit board 31. As a result, transmission of heat generated from the heater 40 to the electric power source part 20 is suppressed.

The second circuit board 32 may be arranged in a position between the top housing 11A and the electric power source part 20, and may extend in a direction orthogonal to the direction along that the first circuit board 31 extends. The power button 13 may be arranged in a position adjacent to the second circuit board 32. When the power button 13 is pressed by a user, a part of the power button 13 may be brought into contact with the second circuit board 32.

For example, each of the first circuit board 31 and the second circuit board 32 may comprise a microprocessor or the like, and control supplying of electric power from the electric power source part 20 to the heater 40. That is, the first circuit board 31 and the second circuit board 32 may control heating of the inhalation article 110 by the heater 40.

The electric power source part 20 comprises an electric power source 21 that is electrically connected to the first circuit board 31 and the second circuit board 32. The electric power source 21 may be a rechargeable battery or a non-rechargeable battery. The electric power source 21 is electrically connected to the heater 40 via at least one of the first circuit board 31 and the second circuit board 32. That is, the electric power source 21 can supply electric power to the heater 40 for appropriately heating the inhalation article 110. Further, as shown in the figure, the electric power source 21 may be adjacently positioned in a direction orthogonal to a longitudinal direction of the heater 40. That is, even if the size of the electric power source 21 is made large, increasing of the longitudinal-direction length of the inhaler 10 can be suppressed.

Further, the inhaler 10 may comprise a terminal 22 which is connectable to an external electric power source (not shown in the figure). The terminal 22 may be connected to a cable of micro-USB (Universal Serial Bus) or the like, for example. In the case that the electric power source 21 is a rechargeable battery, the electric power source 21 can be charged by connecting an external electric power source to the terminal 22 and making current flow from the external electric power source to the electric power source 21. Further, it is possible to make it be able to transmit data relating to operation of the inhaler 10 to an external device by connecting a data transmission cable of micro-USB or the like to the terminal 22.

The heater 40 comprises a heating assembly 41 extending in the longitudinal direction, as shown in the figure. The heating assembly 41 comprises plural cylindrical members and is formed to have a cylindrical body as a whole. The heating assembly 41 is constructed to be able to receive therein a part of the inhalation article 110, and has a function for defining a flow path of air supplied to the inhalation article 110 and a function for heating the inhalation article 110 from an outer periphery or a center thereof.

In the bottom housing 11B, a vent 15 for taking air is formed for making the air flow into the inside of the heating assembly 41. Specifically, the vent 15 is in fluid communication with one end part of the heating assembly 41 (the end part on the left side in Fig. 2). Further, the inhaler 10 comprises a cap 16 attachable/detachable to/from the vent 15. The cap 16 is constructed in such a manner that air can flow into the inside of the heating assembly 41 from the vent 15 even in the state that the cap 15 is being attached to the vent 15, and, for example, the cap 16 may have a through hole, a notch, or the like which is not shown in the figure. By attaching the cap 16 to the vent 15, it becomes possible to prevent a matter generated from the inhalation article 110 inserted into the heating assembly 14 from falling from the vent 15 to the outside of the housing 11. Further, by removing the cap 16, it is possible to clean the inside of the heating assembly 14 or the inner side of the cap 16.

The other end of the heating assembly 14 (the end part on the right side in Fig. 2) is in communication with the opening 12a shown in Fig. 1B. An outer fin 17 having an approximately cylindrical shape is installed between the lid part 14 comprising the opening 12a and the other end part of the heating assembly 41. When the inhalation article 110 is inserted into the inside of the inhaler 10 from the opening 12a of the lid part 14 as shown in Fig. 1B, the inhalation article 110 passes through the outer fin 17 and a part thereof is positioned in the inside of the heating assembly 41. Thus, it is preferable that the outer fin 17 be constructed in such a manner that the size of the opening 12a of the lid part 14 side is larger than the size of the opening on the side of the other end part of the heating assembly 41. That is, it becomes easier to insert the inhalation article 110 from the opening 12a to the inside of the outer fin 17.

In the state that the inhalation article 110 is being inserted into the inhaler 10 from the opening 12a as shown in Fig. 1B, if a user performs puff action applied to the part, which protrudes from the inhaler 10, of the inhalation article 110, i.e., the filter 115 shown in Fig. 2, air flows into the inside of the heating assembly 41 from the vent 15. The taken air passes through the inside of the heating assembly 41 and, together with aerosol generated from the inhalation article 110, arrives at the inside of the mouth of the user. Thus, the side close to the vent 15 of the heating assembly 41 is an upstream side, and the side close to the opening 12a of the heating assembly 41 (the side close to the outer fin 17) is a downstream side.

### (1-4) Construction of Inhaler

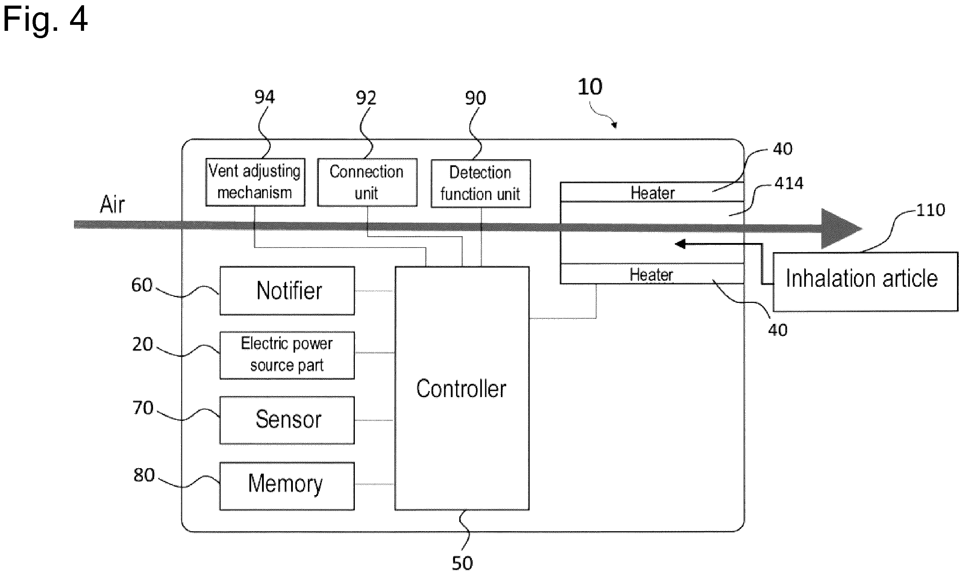

Fig. 4 is a block diagram which schematically shows a construction of the inhaler 10 according to the present embodiment. The inhaler 10 comprises, in addition to the electric power source part 20 and the heater 40 which have been explained above, a controller 50, a notifier 60, a sensor 70, and a memory 80, and they are electrically connected. Further, the inhaler 10 comprises a detection function unit 90, a connection unit 92, and a vent adjusting mechanism 94.

The electric power source part 20 supplies electric power to respective components such as the heater 40, the controller 50, the notifier 60, the sensor 70, the memory 80, the detection function unit 90, the connection unit 92, the vent adjusting mechanism 94, and so on. Especially, as a result of pressing of the power button 13 by a user, electric power is suppled from the electric power source part 20 to the heater 40 for a predetermined period of time, and the heater 40 is activated to heat the inhalation article 110 (aerosol generation base-material). Alternatively, the electric power source part 20 may be constructed in such a manner that it is connected to a different device comprising an electric power source 21.

The inhaler 10 further comprises a holding part 414 which has a concave shape and can receive the inhalation article 110 and hold the filling article 111. The heater 40 may have a shape for heating the inserted and received inhalation article 110 from an outer periphery or a center thereof. That is, the heater 40 can heat a part of the inhalation article 110 which includes a flavor source and is held by the holding part 414, when supply of electric power is received from the electric power source part 20.

The controller 50 is constructed to control the heater 40, the notifier 60, the sensor 70, the memory 80, the detection function unit 90, the connection unit 92, the vent adjusting mechanism 94, and so on. Further, it is constructed to communicate information between it and respective components. The controller 50 may be an electronic circuit module constructed as a microprocessor or a microcomputer. The controller 50 may be constructed to control operation of the inhaler 10, according to computer-executable instructions stored in the memory 80. The controller 50 reads data from the memory 80 and uses the data for control of the inhale 10 as necessary, and stores data in the memory 80 as necessary. Especially, the controller 50 instructs the detection function unit 90, that will be explained later, to detect insertion of the inhalation article 110, for judging the type of a flavor source included in the inhalation article 110.

The notifier 60 operates to provide a user with explicit notification. Especially, it gives notification in a form corresponding to each of results of judgment, with respect to flavor sources, in the controller 50. Specifically, the notifier 60 provides a user with notification in various forms, by light emission, display, vocalization, vibration, or a combination thereof, or the like, as necessary. For example, the notifier 60 may be constructed in such a manner that it comprises one or plural LEDs, and makes the one or plural LEDs emit light having a single or plural colors according to a judged flavor-source type.

The sensor 70 may comprise a pressure sensor for detecting change in the pressure or a flow rate sensor for detecting a flow rate in the air taking-in flow path and/or the aerosol flow path from the vent 15 to the heating assembly 41. Further, the sensor 70 may comprise a weight sensor for detecting the weight of a component in the inhalation article 110. Further, the sensor 70 may be constructed to detect the height of an internal liquid surface in the case that the aerosol source is liquid. Further, the sensor 70 may be constructed to detect an SOC (State of Charge, charge state) of the electric power source part 20, or a discharging state, an integrated current value, a voltage, or the like of the electric power source part 20. The integrated current value may be obtained by using a current integration method, an SOC-OCV (Open Circuit Voltage, open circuit voltage) method, or the like. Further, the sensor 70 may be a manipulation button which can be manipulated by a user, or the like.

Further, the sensor 70 may be a temperature detector constructed to detect temperature of the heater 40 (a load included in the heater 40). For example, the temperature detector may be constructed to detect a value (the value of current flowing through the load in the heater 40, the value of a voltage applied to the load in the heater 40, or the like) that is required when obtaining a resistance value of the load in the heater 40. In the case that the resistance value of the load in the heater 40 is dependent on temperature, the temperature of the heater 40 can be estimated based on the detected resistance value of the load in the heater 40. In a different example, the temperature detector may comprise a temperature sensor for detecting temperature of the heater 40.

The memory 80 is a storage medium such as a ROM (Read Only Memory), a RAM (Random Access Memory), a flash memory, or the like. The memory 80 can store various data relating to operation of the inhaler 10. For example, the memory 80 may store data of a heating profile that has been defined with respect to the heater 40 in advance. Further, the memory 80 may store, in addition to computer-executable instructions, setting data that are necessary for controlling the inhaler 10, and programs such as firmware and so on. For example, the memory 80 may store various data relating to the method for controlling the notifier 60 (modes and so on of light emission, vocalization, vibration, and so on), values detected by the sensor 70, the way of adjustment of the vent adjusting mechanism 94, and so on. Further, the memory 80 stores a program for making the inhaler 10 generally perform operation that will be explained later, and the controller 50 executes the program.

The detection function unit 90 is a component which cooperates with the flavor indication unit 120 installed in the inhalation article 110, and may be constructed by using a sensor element corresponding to the flavor indication unit 120. It is preferable that the detection function unit 90 be arranged in a position, that can be aligned with the flavor indication unit 120, in an inner surface of the concave-shape holding part 414 or a position adjacent thereto. The detection function unit 90 detects, at predetermined timing, a state that the inhalation article 110 is being inserted in the inhaler 10, and, thereafter, interacts with the flavor indication unit 120. As a result of interaction with the flavor indication unit 120, it becomes possible to judge the type of a flavor source in the inhalation article 110.

For example, in the case that the flavor indication unit 120 is constructed by using the tip part, which has a predetermined shape, of the base-material part 110A, it is preferable that the detection function unit 90 be constructed by using a pressure sensor element. The modes of contact between the holding part 414 and the tip parts of the base-material parts 110A, when the inhalation articles 110 are inserted and received by the holding part 414 of the inhaler 10, would be different from one another, according to flavor-source types. For example, when the tip part of the base-material part 110A is brought in contact with the holding part 414 of the inhaler 10 with pressure, the contact area and the pressure thereof in the present case are different from those in other cases. That is, it becomes possible to judge the type of a flavor source in the inhalation article 110 in response to a pressure value detected by the detection function unit 90 which is a pressure sensor element.

In this regard, as explained with reference to Fig. 2, when the inhalation article 110 is inserted in the inhaler 10, the filling article 111 in the inhalation article 110 is held in a position on the holding part 414 side, specifically, on an upstream side close to the vent 15 in the longitudinal direction on the holding part 414 side. In the upstream side of the heating assembly 41, there may be a case that the filling article 111 and/or a generated matter fall to the side of the holding part 414, when action for inserting the inhalation article 110 is being performed and/or when puff action is being performed. Thus, it is preferable that the detection function unit 90 be installed, in an inner surface of the holding part 414 or in a positon close thereto, on the downstream side that is opposite to the upstream side in the longitudinal direction, that is, a position close to the opening 12a for receiving the inhalation article 110. That is, regarding the detection function unit 90, lowering of accuracy of detection thereof due to pollution by the fallen filling article 111 and/or the fallen generated matter can be prevented.

The connection unit 92 is used when communicably connecting the inhaler 10 to an external device. Communication in the subject case may be any of wired communication and wireless communication. In the case of wired communication, it is constructed that data relating to operation of the inhaler 10 is inputted/outputted to/from an external device by connecting a data transmission cable of micro-USB or the like by using an external connection terminal (the terminal 22 in Fig. 3). Especially, it is preferable that it is constructed in such a manner that, when connection to an external device via the terminal 22 is completed, various kinds of setting data and/or firmware of the inhaler 10 stored in the memory 80 can be rewritten based on an instruction from the external device.

For example, it is preferable to construct the inhaler 10 in such a manner that information relating to control modes (light emission, vocalization, vibration, and so on) of the notifier 60 corresponding to flavor-source types, and information relating to setting of heating (the allowed temperature range at the time of heating, time of heating, the number of times of puff actions, and so on) at the time of puff action can be rewritten via the connection unit 92. That is, a user can customize inhalation experience according to preference of the user, and can improve convenience.

On the other hand, regarding examples of the case that communication by the connection unit 92 is wireless communication, they include near field communication using Bluetooth (Registered Trademark: for example, BLE, Bluetooth Low Energy) and so on. In the above case, the connection unit 92 is a communication module. By using a communication module, the inhaler 10 can communicate with an external device via a network. That is, for example, it is possible to transmit, in real time, information of the inhaler 10, such as the flavor-source type and so on, during puff action of a user to an external device via a network.

The vent adjusting mechanism 94 adjusts the size of the opening area of the vent 15 in response to an instruction from the controller 50. For example, the vent adjusting mechanism 94 can adjust the quantity of air flowing into the vent 15 by adjusting the opening area of the vent 15 to correspond to the judged flavor-source type.

For example, in this case, it is assumed that the vent 15 comprises an approximately-semicircular-shape opening. In such a case, it is preferable that the vent adjusting mechanism 94 comprise a small-size motor (not shown in the figure) having a rotating shaft extending, in the longitudinal direction, toward the center of the vent 15, and a flat-plate-shape cover (not shown in the figure) attached to the tip of the rotating shaft in such a manner that it is positioned to be approximately orthogonal to the rotating shaft. By constructing the cover to have a diameter that is the same as that of the vent 15 and a shape that is approximately the same as that of the vent 15, the vent 15 can be closed thereby.

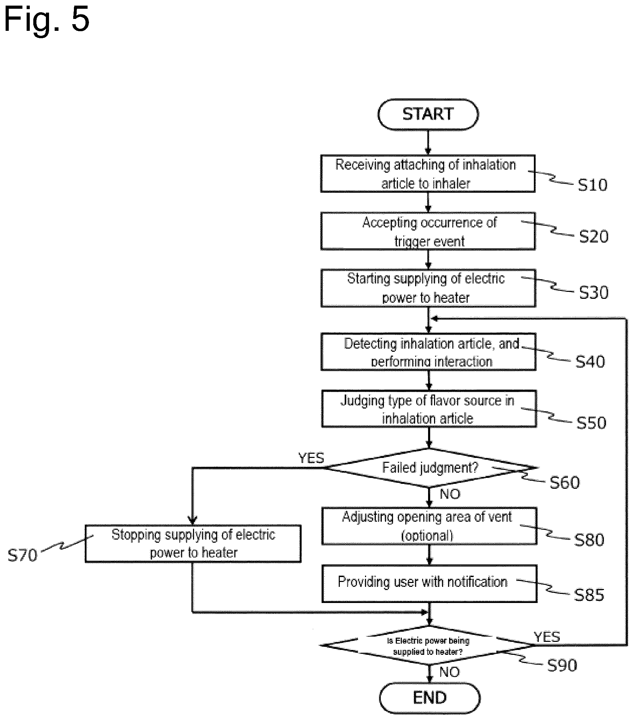

The original basic position of the cover is a position whereat the cover does not close the vent 15. On the other hand, when the small-size motor is rotated and the cover is rotated, for example, 180 degrees, the cover closes the vent 15 by covering the whole of the vent 15. Further, the quantities of rotation of the cover from the original position (0-180 degrees) have been set to correspond to respective flavor-source types and stored in the memory 80 in advance, and, in response to judgment of the flavor-source type, the vent adjusting mechanism 94 makes the cover be rotated by the quantity of rotation relating to the type. That is, it is possible to adjust the opening area of the vent to correspond to the judged flavor-source type (S80 in Fig. 5).

### (1-5) Operation of Inhaler

Fig. 5 is a schematic flow chart of operation of the inhaler 10 according to the present embodiment. By activating the inhaler 10 according to the present embodiment, it becomes possible to provide a puff environment (for example, an atmosphere at the time of puff action) corresponding to a concept of a flavor sucked by a user, and provide further appropriate inhalation experience.

First, in step S10, attaching (inserting) of the inhalation article 110 including a flavor source to the inhaler 10 is received. Next, in step S20, the controller 50 accepts occurrence of a trigger event. In the present embodiment, the trigger event includes pressing of the power button 13 by a user. That is, in step S30, the controller 50 starts supplying of electric power from the electric power source part 20 to the heater 40 in response to detection of pressing of the power button 13.

Regarding next step S40, it is preferable that the step be performed at timing that step S30 is performed. That is, in synchronous with a start of supplying of electric power to the heater 40 (step S30), the controller 50 makes the detection function unit 90 detect the inserted inhalation article 110. Specifically, the detection function unit 90 detects the state that the inhalation article 110 is being inserted into the inhaler 10, and interacts with the flavor indication unit 120 of the inhalation article 110. For example, in the case that the flavor indication unit 120 comprises a tip part, which has a predetermined shape, of the base-material part 110A, and the detection function unit 90 comprises a pressure sensor element, it is preferable to have a construction to detect a pressure vale in the state that the inhalation article 110 and the flavor indication unit 120 are being in contact with each other be detected.

In the present embodiment, in step S40, the pressure sensor element, which is the detection function unit 90, is activated as a result that the power button 13 is pressed in step S20. In other words, if the power button 13 is not pressed, the pressure sensor element is not activated. That is, it is possible to prevent waste in terms of electric power consumption in the inhaler 10, and extend the life of the inhaler 10 (especially, the electric power source part 20). The above matters will result in lowering of frequency of discarding of inhalation articles, and will lead to provision of environmentally friendly inhalers by preventing unnecessary waste of inhaled component sources, so that it is advantageous from the perspective of energy conservation and environmental preservation.

In step S50, the controller 50 judges the flavor-source type according to the pressure value detected as a result of interaction in step S40. Similar to the above-explained example, in the case that the detection function unit 90 comprises a pressure sensor element, a range of pressure value that may be expected with respect to each of flavor-source types may be stored in the memory 80 in advance, and the controller 50 may be constructed to judge the flavor-source type by determining a range in which the detected pressure value is included.

Regarding step S50, for example, in the case that the inhalation article 110 is not correctly inserted in the inhaler 10, or in the case that a non-regular inhalation article, that is not a genuine product, or a foreign matter is inserted in the inhaler 10, or the like, the flavor-source type is not judged normally. That is, judgment will end in failure. Accordingly, in next step S60, the controller 50 outputs a result representing whether judgment of the type of the flavor source in the inhalation article 110 has been completed successfully or has ended in failure, and, thereafter, performs operation corresponding to the result.

If it is judged in step S60 that the controller 50 has failed in judgment of the flavor-source type (YES), the controller 50 stops supplying of electric power from the electric power source part 20 to the heater 40 in step S70. That is, according to this step S70, an event that is so-called "heating without an object to be heated," wherein heating by the heater 40 is performed during a state that the inhalation article 110 is not normally being inserted, can be prevented, or unfair use of a non-regular inhalation article, that is not a genuine product, can be prevented.

On the other hand, if it is judged in step S60 that judgment of the flavor-source type did not end in failure (NO), the controller 50 makes the vent adjusting mechanism 94 adjust the opening area of the vent 15 according to the judged flavor-source type in step S80, to adjust the quantity of air taken into the inside of the inhaler 10. In this manner, by making it possible to adjust the quantity of air, it becomes possible to adjust the quantity of generation of aerosol at the time of puff action by a user, so that it becomes possible to supply flavor appropriately according to the flavor-source type. That is, it becomes possible to provide a user with higher-quality inhalation experience. It should be reminded that this step S80 may be optional.

Next, in step S85, the controller 50 makes the notifier 60 perform notification corresponding to the judged flavor-source type. For example, in the case that it is judged that the flavor-source type is a usual regular flavor source, it makes an LED emit light having a red color that represents a basic color of a puff action. On the other hand, in the case that it is judged that the flavor-source type is a menthol flavor source, it makes an LED emit light having a pale blue color that makes a user recall cool sensation of menthol. Further, in the case that it is judged that the flavor-source type is a citrus flavor source, it makes an LED emit light having a lemon color or an orange color that makes a user recall a citrus.

In this manner, by performing notification corresponding to each judged flavor-source type, it becomes possible to make a user visually recognize the type of the flavor source which is being sucked by the user, and, through this process, it becomes possible to provide the use with higher-quality inhalation experience.

Even in the case that notification corresponding to the judged flavor-source type has been performed once by the notifier 60 in step S85, it may be preferable that the controller 50 periodically repeat operation for judging the flavor-source type, while electric power is being supplied to the heater 40. That is, in step S90, the controller 50 judges whether supplying of electric power to the heater 40 is being continued. In the case that electric power is being supplied (YES), the process continues to return to the operation of interaction with the inhalation article 110 (step S40), and judging of the flavor-source type (S50) is periodically repeated. Further, regarding the following control operation (S60-S90), they are also periodically repeated.

By periodically performing operation for judging flavor-source type as explained above, it becomes possible to quickly detect abnormal states such as a state that an inhalation article 110 is removed from the inhaler 10 after the inhalation article 110 is inserted into the inhaler 10 once, a state that the inhalation article 110 has slid from a normal positon to an abnormal position in the holding part 414, and so on. According to the present embodiment, even in the case that an abnormal state is detected, stopping of supplying of electric power to the heater 40 is performed in above-explained step S70 as part of normal operation control. That is, unnecessary supplying of electric power can be prevented, and the life of the inhaler 10 (especially, the electric power source part 20) can be further extended. Further, provision of bad inhalation experience can be prevented.

The series of control operation in step S10 to step S90 is terminated after a stop of supplying of electric power to the heater 40 is confirmed in step S90 (NO).

### (1-6) Modification Examples

### (1-6-1) Modification Example of Flavor Indication Unit

It is explained in the above description that it is constructed that the flavor indication unit 120 of the inhalation article 110 is formed by making the shape and/or the material of the inhalation article 110 correspond to a flavor-source type, and that the modes of contact between respective flavor indication units 120 and the detection function unit 90 in the inhaler 10 are different from one another according to respective flavor-source types. However, the construction of the flavor indication unit 120 is not limited to that explained above, and it may be constructed as a part which includes any information and/or any mechanism if they/it can represent a flavor-source type.

For example, the flavor indication unit 120 may be constructed as a material part which is attached to an outer periphery of the inhalation article 110 and includes aroma components, and, in such a case, different aromas corresponding to different flavor-source types are generated. In a different embodiment, the flavor indication unit 120 may be constructed as a part which is attached to an outer periphery of the inhalation article 110 and includes a one-dimensional or two-dimensional barcode. In a further different example, the flavor indication unit 120 may be constructed as a contact-type or non-contact-type IC chip which is attached to a predetermined part on an outer periphery of the inhalation article 110. It is possible to specify a flavor-source type from information given by the barcode or the IC chip.

Each of a material part including an aroma component, a barcode, and an IC chip, such as those explained above, may be constructed, for example, as a material such as a seal constructed to be attached to a predetermined part of the first rolling paper 112 and/or the second rolling paper 112 and 113, or the like, or may be constructed directly in a predetermined part. Especially, in the case that a barcode or an IC chip is adopted, it is possible to include various kinds of information therein, in addition to information of the flavor-source. For example, manufacturing information such as the manufacturing number, the date of manufacture, the expiration date, and so on, puff information such as the range of temperature at the time of heating, the total time of heating, the maximum number of times of puff, and so on, and other information may be included.

### (1-6-2) Modification Example of Detection Function Unit

The case explained in the above description is that the flavor indication unit 120 comprises the tip part, which has a predetermined shape, of the base-material part 110A, and, for corresponding thereto, the detection function unit 90 of the inhaler 10 comprises a pressure sensor element. However, the construction of the detection function unit 90 is not limited to that explained above, and it may be constructed as that having a construction corresponding to the above-explained flavor indication unit 120.

For example, in the case that the flavor indication unit 120 is constructed as the above-explained material part including aroma components, it is preferable that the detection function unit 90 comprise an odor sensor component. That is, the type of a flavor source in the inhalation article 110 can be judged according to the kind of odor sensed by the odor sensor element. In a different example, in the case that the flavor indication unit 120 is constructed as the above-explained part including a one-dimensional or two-dimensional barcode, it is preferable that the detection function unit 90 comprise a barcode reader. That is, based on the value read by the barcode reader, the flavor-source type can be judged, and, further, the inhalation article 110 can be identified.

In a further different example, in the case that the flavor indication unit 120 is constructed as a contact-type IC chip, it is preferable that the detection function unit 90 comprise a contact detection electrode. Similarly, in the case that the flavor indication unit 120 is constructed as a non-contact-type (especially, a proximity-type) IC chip or IC tag, it is preferable that the detection function unit 90 comprise a reader (including a reader/writer) compliant with a short-distance communication standard. That is, in response to contact of the IC chip with the contact detection electrode, and in response to establishment of short-distance wireless communication, the flavor-source type can be judged, and, further, the inhalation article 110 can be identified.

### (1-6-3) Modification Example of Notifier

It is explained in the above description that notification by the notifier 60 in the inhaler 10 (S85 in Fig. 5) comprises emission of light that is performed by one or plural LEDs and corresponds to each judged flavor-source type. However, the mode of notification is not limited to that explained above, and any mode of operation that can provide a user with an explicit notification can be adopted, and it can be realized by light emission, display, vocalization, vibration, or a combination thereof, or the like. Thus, a flexible mode of notification, that is given to a user, can be realized.

For example, the notifier 60 may comprise one or plural vibrators, and may be constructed to generate vibration that has one or plural vibration types corresponding to judged flavor-source types. For example, in the case that the flavor-source type is a usual regular flavor source, it is set to a basic number of times of vibration and a vibration frequency, with respect to a puff action; in the case that the flavor-source type is a menthol flavor source, it is set to a long vibration frequency; in the case that the flavor-source type is a citrus flavor source, it is set to a short vibration frequency and an increased number of times of vibration, and so on.

In a different example, the notifier 60 may comprise one or plural speakers, and may be constructed to generate sounds corresponding to judged flavor-source types. For example, in the case that the flavor-source type is a usual regular flavor source, a basic sound is generated with respect to a puff action; in the case that the flavor-source type is a menthol flavor source, a high-pitch sound is generated; in the case that the flavor-source type is a citrus flavor source, a low-pitch sound is generated, and so on. In a different construction, the notifier 60 may comprise one or plural displays, and displaying on the display(s) is performed in response to the judged flavor-source type.

### (1-6-4) Modification Example of Vent Adjusting Mechanism

It is explained in the above description that it is constructed that the vent adjusting mechanism 94 of the inhaler 10 comprises a small-size rotating motor and a lid, and the vent 15, which is an approximately-semicircular-shape opening, is closed as a result that the lid is rotated by a predetermined quantity of rotation. However, the vent adjusting mechanism 94 is not limited as explained above, and any mechanism which allows adjustment of the diameter of the vent 15 according to the flavor-source type can be adopted.

### (1-6-5) Modification Example of Trigger Event

It is explained in the above description that a trigger event for making the inhaler 10 start supplying of electric power to the heater 40 (step S30) and/or for performing detection of the inhalation article 110 (step S40) is an action of pressing of the power button 13 (step S20). In more detail, it is constructed that the inhaler 10 performs, in synchronous with a start of supplying of electric power to the heater 40 that begins in response to pressing of the power button 13, detection of the inhalation article 110, interaction with the inhalation article 110, and judgment of the flavor-source type (steps S20-S50 in Fig. 5). However, processes for detecting the inhalation article 110 and judging the flavor-source type such as those in steps S40, S50, and so on are those that are not necessarily performed in synchronous with a start of supplying of electric power to the heater 40, and they may be constructed in such a manner that they are performed in response to different trigger events. As a result, it becomes possible to flexibly perform operation for judging the flavor-source type.

For example, a different trigger event is detection of a puff action performed by a user. That is, after a start of supplying of electric power to the heater 40, if a puff action performed by a user is detected, operation for detecting the inhalation article 110 and judging the flavor-source type may be performed in response to the detection of the puff action. In this regard, for effectively detecting a puff action by a user, it is preferable to construct it to use a puff sensor such as a microphone condenser that is to be included in the sensor 70 in the inhaler 10.

### (1-6-6) Modification Example of Operation of Inhaler

It may not be required to perform the processes (S10-S90) of operation of the inhaler 10 shown in Fig. 5 in the above-explained order. For example, instead of performing the operation in above-explained step S30 before performing operation of above-explained respective steps S40 and S50, the operation may be performed after performing operation of above-explained respective steps S40 and S50. In more detail, it is preferable to perform a process of interaction with the inhalation article 110 (step S40) and a process of judgment of the flavor-source type (step S50), and, only when judgment is successfully completed (step S60: NO), perform the process of starting of supply of electric power from the electric power source part 20 to the heater 40 in step S30. In the case of that judgment has ended in failure (step S60: YES), it is preferable to construct it to perform, in place of step S70, control operation for disallowing supplying of electric power from the electric power source part 20 to the heater 40.

As explained above, in the modification example of the present embodiment, by adopting the construction that supplying of electric power to the heater 40 starts only when judgment of the flavor-source type is successfully completed, waste in terms of electric power consumption in the inhaler 10 can be prevented. That is, the life of the inhaler 10 (especially, the electric power source part 20) can be extended. The above matters will result in lowering of frequency of discarding of inhalation articles, and will lead to provision of environmentally friendly inhalers by preventing unnecessary waste of inhaled component sources, so that it is advantageous from the perspective of energy conservation and environmental preservation.

### (1-7) Application Examples

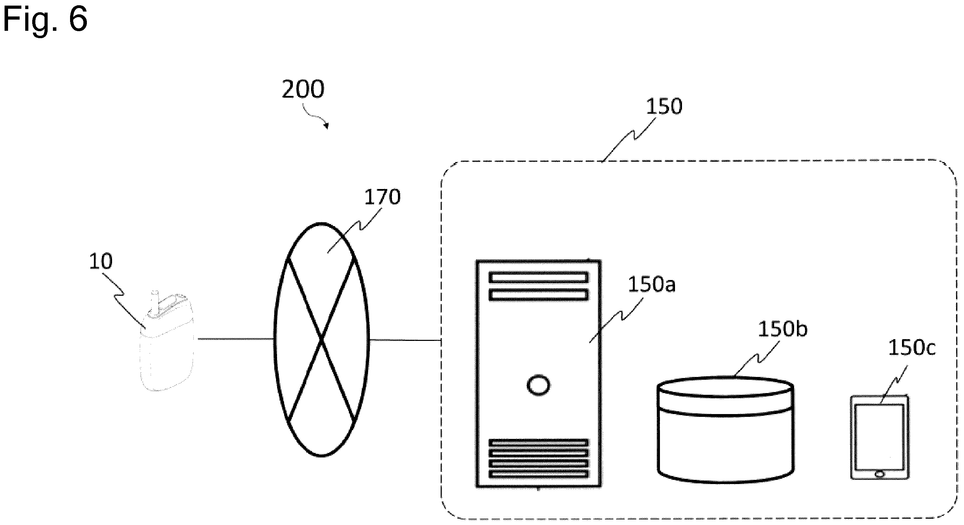

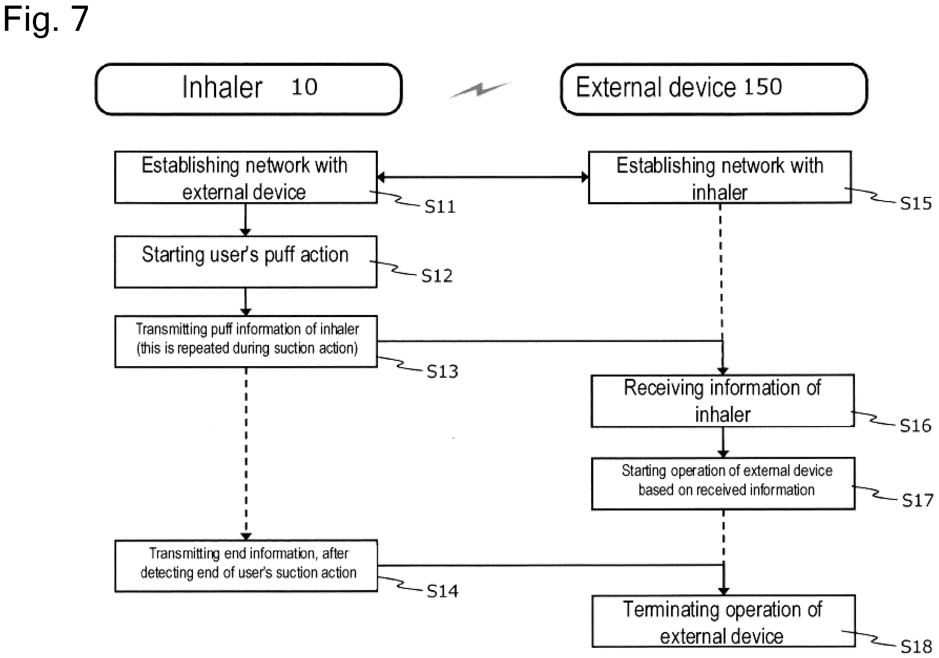

Next, an application example of the inhaler 10 of the present embodiment will be explained. Fig. 6 schematically shows an example of application of the inhaler 10 according to the present embodiment to an inhalation experience providing system 200. In addition, Fig. 7 is a sequence diagram which schematically shows operation of the inhalation experience providing system 200.

The inhalation experience providing system 200 comprises the inhaler 10, a network 170, and an external device 150. It is preferable to construct the inhaler 10 in such a manner that it transmits, via its connection unit 92 (a communication module), information obtained by the controller 50 and/or information stored in the memory 80 to the external device 150 through the network 170. For example, information of the flavor-source type judged by the controller 50 is transmitted in real time to the external device 150.

It is preferable that the network 170 be constructed as a near field wireless communication network conforming to Bluetooth (Registered Trademark) or the like, a WiFi network conforming to IEEE 802.11 standard, or a wireless communication network in the Internet for example.

The external device 150 comprises processors such as a CPU (Central Processing Unit) and/or a GPU (Graphical Processing Unit) and a memory (they are not shown in the figure), and a program stored in the memory is read into the CPU, the GPU, or the like in a computer. By using them, various kinds of information processes are performed. For example, the external device 150 may be a terminal possessed by a user, such as a home computer 150a, a smart speaker 150b, a smartphone 150c (or a tablet), or the like. Also, the external device 150 may be a household electric device, such as an aroma generating device, an air cleaner, an air conditioner, a refrigerator, or the like which is installed in a room, wherein a user performs a puff action, and can be connected to the network 170 such as a domestic network, the Internet, or the like.

In the sequence relating to the inhalation experience providing system 200 in Fig. 7, interactive communication between the inhaler 10 and the external device 150 is performed. First, in steps S11 and S15, connection by the network 170 between the inhaler 10 and the external device 150 is established. For example, in the case that the network 170 is that conforming to Bluetooth (Registered Trademark), network connection between the inhaler 10 and the external device 150 is established by performing a pairing process.

Thereafter, in step S12, the inhaler 10 accepts a start of a puff action by a user. That is, a user inserts the inhalation article 110 into the inhaler 10, and presses the power button 13 to start a puff action. At that time, the inhaler 10 performs judgment of the type of a flavor source (step S50 in Fig. 5). During a puff action by a user, the inhaler 10 repeatedly transmits, periodically, information of the inhalation article 110, including the flavor-source type, to the external device 150, in step S13.

In relation to step S15, in step S16, the external device 150 periodically receives information of the inhalation article 110 including information of the flavor-source type. Next, in step S17, the external device 150 operates itself in a desired mode, based on the information received in step S16.

For example, in the case that the external device 150 is a home smart speaker 150b, the external device 150 may be constructed to generate sounds corresponding to types of flavor sources. Especially, it is preferable to construct it in such a manner that music files that have been registered with the external device 150 in advance by a user are reproduced. Also, in the case that the external device 150 is an aroma generating device, it may be constructed to generate aromas corresponding to types of flavor sources. In the external device 150, various kinds of information, other than the flavor-source types, can be used, and various kinds of operation can be implemented.

Operation of the external device 150 is continued, until the inhaler 10 detects an end of the user's puff action in step S14 and the inhaler 10 stops its operation in step S18 in response to detection of the end.

As explained above, the inhalation experience providing system 200 can provide a user with a higher-quality inhalation experience, for example, by operating the external device 150 to improve the surrounding environment according to the type of a flavor source. Especially, it is possible to provide a room, wherein a user performs puff action, as a higher-quality space for puff action.

### < Second Embodiment >

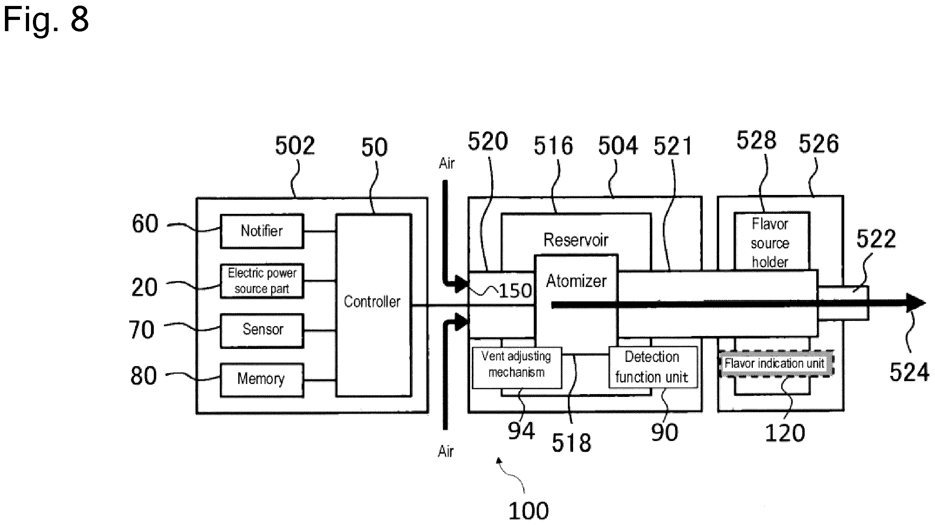

In the following description, an inhaler 100 according to a second embodiment will be explained with reference to Fig. 8. It should be reminded that, regarding the constructions and the functions that have been explained, substantially the same reference symbols that have been used are assigned thereto, and explanation thereof will be omitted. In the inhaler 100 in the present embodiment, the electric power source of the inhaler 100 is turned on when a user's puff action is detected. That is, in response to detection of a user's puff action, supplying of electric power is started and aerosol is generated.

### (2-1) Construction, Operation, and Modification Examples of Inhaler

Fig. 8 is a schematic block diagram of a construction of an inhaler 100 according to a second embodiment. As shown in Fig. 8, in the present embodiment, the inhaler 100 comprises a first member 502, a second member 504, and a third member 526, and is constructed by attaching them together. Specifically, the second member 504 is attached to the first member 502 in an attachable/detachable manner, and the third member 526 is attached to the second member 504 in an attachable/detachable manner. Further, in the present embodiment, the second member 504 is an inhalation article including an aerosol source, and the third member 526 is an inhalation article including a flavor source.

For example, the first member 502 may be an electric power source unit, and may comprise a controller 50, a notifier 60, an electric power source part 20, a sensor 70, a memory 80, and a connection unit 92 (not shown in the figure) which are electrically connected. The inhalation article (including an aerosol source), which is the second member 504, may be constructed as a cartridge, and may comprise a reservoir 516, an atomizer 518, an air taking-in flow path 520, and an aerosol flow path 521, and a detection function unit 90 and a vent adjusting mechanism 94. The inhalation article (including a flavor source), which is the third member 526, may be constructed as a capsule, and may comprise a flavor source holder 528 and a suction opening part 522. In the case that the inhaler 100 is an electronic cigarette, a fragrance-inhaling-taste component included in tobacco may be included in the flavor source holder 528.

### (2-1-1) Construction of Electric Power Source Unit

Regarding the electric power source which is the first member 502, the electric power source part 20 supplies electric power to the atomizer 518 for atomizing the aerosol source in response to puff action of a user (hereinafter, the "heater" explained in relation to the first embodiment may be referred to as an "atomizer" in the second embodiment).

The controller 50 instructs the detection function unit 90 to detect, at predetermined timing, attaching of the third member 526, and judges the type of the flavor source included in the third member 526. As a result, it becomes possible to provide a puff environment (for example, an atmosphere at the time of puff action) corresponding to a concept of a flavor sucked by a user, and provide further appropriate inhalation experience, through operation of transition.

Especially, it is preferable to construct the controller 50 to judge the flavor-source type in response to detection of a user's puff action. By the above construction, a start of operation for judging the flavor-source type is synchronized with a start of supplying of electric power to the atomizer 518. In other words, if a puff action is not detected, the operation for judging the flavor-source type is not performed. That is, it is possible to prevent waste in terms of electric power consumption in the inhaler 100, and extend the life of the inhaler 100 (especially, the electric power source part 20). The above matters will result in lowering of frequency of discarding of inhalation articles, and will lead to provision of environmentally friendly inhalers by preventing unnecessary waste of inhaled component sources, so that it is advantageous from the perspective of energy conservation and environmental preservation.

In an alternative example, the controller 50 may be constructed to judge the flavor-source type in response to pressing of a predetermined manipulation button (for example, a power button (which is not shown in the figure)) by a user. By the above construction, operation for judging the type of a flavor source can be implemented flexibly.

Also, it is possible to adopt the construction that supplying of electric power from the electric power source part 20 to the atomizer 518 is started only when judging of the flavor-source type is successfully completed. Further, it is possible to adopt the construction that supplying of electric power is not performed in the case that judgment has ended in failure. As a result, it becomes possible to prevent waste in terms of electric power consumption in the inhaler 100. That is, the life of the inhaler 100 (especially, the electric power source part 20) can be extended further. The above matters will result in lowering of frequency of discarding of inhalation articles, and will lead to provision of environmentally friendly inhalers by preventing unnecessary waste of inhaled component sources, so that it is advantageous from the perspective of energy conservation and environmental preservation.

Further, it is preferable to adopt the construction that the controller 50 periodically repeats operation for judging the flavor-source type during supplying of electric power from the electric power source part 20 to the atomizer 518. As a result, it becomes possible to quickly detect abnormal states such as a state that a third member 256 is removed from the inhaler 100 after the third member 256 is inserted into the inhaler 10 once. Further, unnecessary supplying of electric power can be prevented, and the life of the inhaler 100 (especially, the electric power source part 20) can be further extended. Further, provision of bad inhalation experience can be prevented.

In addition, it is preferable to adopt the construction that the controller 50 immediately stops supplying of electric power from the electric power source part 20 to the atomizer 518 in the case that the controller 50 has failed in judgment of the flavor-source type. That is, in the case that judgment of the flavor-source type has ended in failure due to the state that a third member 526 is not being inserted in a regular manner or the like, an event that is so-called "heating without an object to be heated," that occurs when heating/atomizing is performed during the above state, can be prevented, or unfair use of a non-regular inhalation article, that is not a genuine product, can be prevented.

The notifier 60 operates to provide a user with explicit notification. Especially, it gives notification in a form corresponding to each of results of judgment with respect to flavor sources. For example, the notifier 60 may be constructed in such a manner that it comprises one or plural LEDs, and makes the one or plural LEDs emit light having a single or plural colors according to a judged flavor-source type. Also, the notifier 60 may comprise one or plural vibrators, and may be constructed to generate vibration that has one or plural vibration types corresponding to judged flavor-source types. Further, the notifier 60 may comprise one or plural speakers, and may be constructed to generate sounds corresponding to judged flavor-source types. Thus, a flexible mode of notification, that is given to a user, can be realized.

The sensor 70 comprises various types of sensors. For example, it may be constructed to include a puff sensor such as a microphone condenser. As a result, puff action of a user can be detected effectively.

Similar to the case of the first embodiment, the connection unit 92 (not shown in the figure) may be an external connection terminal, and it is preferable to adopt the construction that, when it is connected to an external input device, various kinds of setting data and/or firmware of the inhaler 100, that have been stored in the memory 80 are rewritable based on instructions from the external input device. In a different case, the connection unit 92 may be a communication module, and it is preferable to adopt the construction that, during inhalation action by a user, information of the third member 526, such as the flavor-source type, and so on is transmitted to an external device via a network in real time. As a result, it becomes possible to provide a puff environment (for example, an atmosphere at the time of puff action) corresponding to a concept of a flavor sucked by a user, and provide further appropriate inhalation experience, through operation of transition.

### (2-1-2) Construction of Cartridge

Regarding the cartridge which is the second member 504 (the inhalation article (including an aerosol source)), the reservoir 516 holds an aerosol source. For example, the reservoir 516 comprises fibrous or porous material, and holds an aerosol source, which is in the form of liquid, by use of spaces between fibers or pores in the porous material. For example, cotton or glass fibers, or tobacco raw material, or the like, may be used as the above-explained fibrous or porous material. The reservoir 516 may be constructed as a tank for storing liquid. The reservoir 516 may have a construction which allows replenishment of a consumed aerosol source. In a different case, the reservoir 516 may be constructed in such a manner that the reservoir 516 itself is allowed to be replaced when the aerosol source is exhausted. Further, the aerosol source is not limited to that in a liquid form, and it may be solid. In the case that the aerosol source is solid, the reservoir 516 may be a hollow container which does not use fibrous or porous material, for example.

The atomizer 518 is constructed to generate aerosol from an aerosol source. Specifically, the atomizer 518 generates aerosol by atomizing or vaporizing an aerosol source. In the case that the inhaler 100 is a medical inhaler such as a nebulizer or the like, the atomizer 518 generates aerosol by atomizing or vaporizing an aerosol source including a medicine. When a puff action is detected by the sensor 70, the atomizer 518 generates aerosol by receiving supply of electric power from the electric power source part 20. For example, a wick (not shown in the figure) may be installed for connection between the reservoir 516 and the atomizer 518. In the above case, a part of the wick extends to the inside of the reservoir 516 and is in contact with the aerosol source. The other part of the wick extends toward the atomizer 518. The aerosol source is sent from the reservoir 516 to the atomizer 518 by capillary effect in the wick. For example, the atomizer 518 comprises a heater which is electrically connected to the electric power source part 20. The heater is arranged to be in contact with or to be positioned close to the wick. When a puff action is detected, the controller 50 controls the heater in the atomizer 518 to heat an aerosol source, which is conveyed via the wick, to thereby atomize the aerosol source. The other example of the atomizer 518 may be an ultrasonic-type atomizer which atomizes the aerosol source by ultrasonic vibration.

Regarding the cartridge which is the second member 504, a vent 15a for taking air for making the air flow into the inside of the reservoir 516 is formed therein. Further, the air taking-in flow path 520 connected to the vent 15a is connected to the atomizer 518, and leads to the outside of the inhaler 100. The aerosol generated in the atomizer 518 is mixed with air that is taken via the air taking-in flow path 520. The fluid mixture comprising the aerosol and the air is sent to the aerosol flow path 521, as shown by an arrow 524. The aerosol flow path 521 has a tubular structure, which extends through the second member 504 and the third member 526, for sending the fluid mixture comprising the air and the aerosol, that is generated in the atomizer 518, to the suction opening part 522 in the third member 526.

The vent adjusting mechanism 94 is installed in relation to the vent 15a. In the inhaler 100, when the second member 504 (the cartridge) is attached to the first member 502 (the electric power source unit), the vent adjusting mechanism 94 is electrically connected to the controller 50 in the electric power source unit 502. Thereafter, the controller 50 makes the vent adjusting mechanism 94 adjust the size of the opening area of the vent 15a according to the flavor-source type judged via the detection function unit 90. As a result, the quantity of air taken into the inside of the inhaler 100 can be adjusted. By making the quantity of air adjustable, it becomes possible to adjust the quantity of generation of aerosol when a puff action is being performed by a user, so that it becomes possible to supply appropriate flavor according to the type of a flavor source. That is, it becomes possible to provide a user with higher-quality experience.

Regarding the detection function unit 90, it is preferable that it is arranged in a position that can be aligned with the flavor indication unit 120 installed in the third member 526, when the third member 526 is attached to the second member 504. The detection function unit 90 is electrically connected to the controller 50 in the first member 502. The detection function unit 90 detects, at predetermined timing, a state that the third member 526 is being attached to the main body of the inhaler 100 (the first member 502 to which the second member 504 has been attached), and, thereafter, interacts with the flavor indication unit 120. As a result of interaction with the flavor indication unit 120, it becomes possible to judge the type of the flavor source in the third member 526 (the inhalation article or the capsule including the flavor source).

### (2-1-3) Construction of Capsule

Regarding the capsule which is the third member 526 (the inhalation article (including flavor source)), the flavor source holder 528 is a component for adding flavor to aerosol. The flavor source holder 528 is positioned in the middle of the aerosol flow path 521. The fluid mixture comprising the air and the aerosol generated by the atomizer 518 (it should be reminded that the fluid mixture may simply be referred to as aerosol, hereinafter) flows to the suction opening part 522 through the aerosol flow path 521. In this manner, in the point of view of the flow of the aerosol, the flavor source holder 528 is arranged in a position downstream the atomizer 518. In other words, in the aerosol flow path 521, the position of the flavor source holder 528 is closer to the suction opening part 522 than the position of the atomizer 518. Thus, the aerosol generated in the atomizer 518 passes through the flavor source holder 528 and thereafter arrives at the suction opening part 522. When the aerosol passes through the flavor source holder 528, fragrance-inhaling-taste components included in the flavor source holder 528 are added to the aerosol.

For example, in the case that the inhaler 100 is an electronic cigarette, the flavor source holder 528 may be that which originates from tobacco, such as shredded tobacco, a product which is made by processing tobacco raw material to have a granular form, a sheet form, or a powder form, or the like. Further, flavor source holder 528 may be that which does not originate from tobacco, such as a product made by use of a plant other than tobacco (for example, mint, a herb, and so on). For example, flavor source holder 528 comprises a nicotine component. The flavor source holder 528 may comprise a flavor component such as menthol or the like. In addition to the flavor source holder 528, the reservoir 116 may also have a material comprising a fragrance-inhaling-taste component. For example, the inhaler 100 may be constructed in such a manner that the flavor source holder 528 holds flavor material which originates from tobacco and the reservoir 516 includes flavor material which does not originate from tobacco.

Regarding the third member 526 (the inhalation article or the capsule (including the flavor source)), the flavor indication unit 120 is installed, for example, on a surface of an outer periphery of the third member 526. The flavor indication unit 120 shows, at least, the type of a flavor source included in the flavor source holder 528. For example, the part of the flavor indication unit 120 is constructed to have a shape or a material according to a flavor-source type. That is, for example, it is possible to construct it in such a manner that the modes of contact (for example, the areas of contact) between the detection function unit 90 and the flavor indication units 120, when the third members 526 are attached to the second member 504 (the inhalation article or the cartridge (including the aerosol source), are set to be different from each other and correspond to respective flavor-source types. Any arrangement of the flavor indication unit 120 may be acceptable if the flavor indication unit 120 is positioned in a position that can be aligned with the position of the detection function unit 90 in the second member 504.

### (2-2) Application Examples

An application example of the inhaler 100 according to the present embodiment is generally similar to the inhalation experience providing system 200 explained in relation to the first embodiment. Specifically, in the present embodiment, when accepting a start of puff action of a user in step S12, judging of the flavor-source type is performed in the inhaler 10 without requiring pressing of the power button 13.

That is, the inhalation experience providing system according to the present embodiment can provide a user with higher-quality inhalation experience, for example, by operating an external device for improving the surrounding environment according to the type of a flavor source. Especially, it is possible to provide a room, wherein a user performs puff action, as a higher-quality space for puff action.

### < Other Embodiments >

In the above description, inhalers, inhalation experience providing systems, and methods according to some embodiments have been explained with reference to the figures. It will be understood that the present disclosure may be implemented as programs for making a processor execute methods for operating an inhaler when the programs are executed by the processor, or a computer-readable storage medium storing the above programs.

In the above description, embodiments of the present disclosure have been explained together with their modification examples and application modes; and, in this regard, it should be understood that they are mere examples, and they are not those limiting the scope of the present disclosure. It should be understood that change, addition, and modification with respect to the embodiments can be performed appropriately, without departing from the gist and the scope of the present disclosure. The scope of the present disclosure should not be limited by any of the above-explained embodiments, and should be limited by the claims and equivalents thereof only.

### REFERENCE SIGNS LIST

10, 100 ... Inhaler: 11 (11A, 11B) ... Housing: 12 ... Cover: 12a ... Opening: 13 ... Power button: 14 ... Lid part: 110 ... Inhalation article (Aerosol generation base-material): 110A ... Base-material part: 110B, 522 ... Suction opening part: 111 ... filling article: 112, 113 ... Rolling paper: 114 ... Paper tube part: 115 ... Filter: 116 ... Hollow segment part: 120 ... Flavor indication unit: 15, 15a ... Vent: 16 ... Cap: 17 ... Outer fin: 20 ... Electric power source part: 21 ... Electric power source: 22 ... Terminal: 30 ... Circuit unit: 31, 32 ... Circuit board: 40 ... Heater: 41 ... Heating assembly: 414 ... Holding part: 50 ... Controller: 60 ... Notifier: 70 ... Sensor: 80 ... Memory: 90 ... Detection function unit: 92 ... Connection unit: 94 ... Vent adjusting mechanism: 502 ... First member (Electric power source unit): 504 ... Second member (Cartridge / Aerosol-source-contained inhalation article): 526 ... Third member (Capsule / Flavor-source-contained inhalation article): 516 ... Reservoir: 518 ... Atomizer (Heater): 520 ... Air taking-in flow path: 521 ... Aerosol flow path: 528 ... Flavor source holder

## Claims

1. An inhaler to which an inhalation article including a flavor source is detachably attached, wherein the inhalation article comprises a flavor indication unit which represents at least the type of the flavor source, and the inhaler comprising:
a holder for holding the flavor source;
a detection function unit which detects the inhalation article and interacts with the flavor indication unit;
a controller which judges, through the detection function unit, the type of the flavor source; and
a notifier which performs notification in a mode corresponding to a result of judgment performed by the controller.

2. The inhaler as recited in Claim 1, wherein the mode comprises emission of light corresponding to the judged flavor-source type.

3. The inhaler as recited in Claim 1 or 2, wherein the mode comprises generation of vibration corresponding to the judged flavor-source type.

4. The inhaler as recited in any one of Claims 1-3, wherein the mode comprises generation of a sound corresponding to the judged flavor-source type.

5. The inhaler as recited in any one of Claims 1-4, further comprising:
a vent for making air flow therein, and a vent adjusting mechanism which can adjust an opening area of the vent; wherein
the controller is configured to make the vent adjusting mechanism adjust the opening area of the vent based on the judged flavor-source type.

6. The inhaler as recited in any one of Claims 1-5, further comprising:
a power button; wherein
the controller is configured to judge the flavor-source type in response to pressing of the power button by a user.

7. The inhaler as recited in any one of Claims 1-5, further comprising:
a sensor which can detect puff action; wherein
the controller is configured to judge the flavor-source type in response to detection of puff action performed by a user.

8. The inhaler as recited in any one of Claims 1-7, further comprising:
an electric power source part and a heater; wherein
the controller is configured to periodically judge the flavor-source type, during the time when electric power is being supplied from the electric power source part to the heater.

9. The inhaler as recited in Claim 8, wherein the controller is configured to stop supplying of electric power from the electric power source part to the heater when the judgment has ended in failure.

10. The inhaler as recited in any one of Claims 1-9, wherein, in the inhaler, the detection function unit is installed in a position close to an opening into which the inhalation article is inserted.

11. The inhaler as recited in any one of Claims 1-10, further comprising:
an external connection terminal; wherein
the mode is changed based on an instruction from an external input device connected via the external connection terminal.

12. The inhaler as recited in any one of Claims 1-11, further comprising:
a communication module; wherein
the communication module is configured to transmit information of the judged flavor-source type to a network.

13. An inhalation experience providing system comprising the inhaler according to Claim 12, comprising:
an external device which is connected to the inhaler via the network; wherein
when information of the flavor-source type is received by the external device, the external device is configured to generate an aroma corresponding to the received flavor-source type.

14. An inhalation experience providing system comprising the inhaler according to Claim 12, comprising:
an external device which is connected to the inhaler via the network; wherein
when information of the flavor-source type is received by the external device, the external device is configured to generate a sound corresponding to the received flavor-source type.

15. A method for operating an inhaler, wherein an inhalation article including a flavor source is attached to the inhaler, and the method comprising:
detecting the inhalation article, and interacting with a flavor indication unit which is provided in the inhalation article and represents at least the type of the flavor source;
judging, according to the interaction, the type of the flavor source; and
performing notification in a predetermined mode corresponding to the judged flavor-source type.

16. The method as recited in Claim 15 further comprising:
accepting pressing, by a user, of a power button; wherein
the detecting and interacting step is performed in response to the accepting step.

17. The method as recited in Claim 15 further comprising:
detecting puff action performed by a user; wherein
the judging step is performed in response to the detecting step.

18. The method as recited in any one of Claims 15-17, wherein the judging step is periodically performed during the time when electric power is supplied to a heater from an electric power source part included in the inhaler.

19. The method as recited in Claim 18 further comprising stopping supplying of electric power to the heater from the electric power source part, in the case that the judgment has ended in failure.

20. A program which makes the inhaler perform the method recited in any one of Claims 15-19.
